# EUROPEAN PATENT APPLICATION

(11) **EP 3 974 453 A2**
(43) Date of publication of application: **30.03.2022**
(21) Application number: 21183531.9
(22) Date of filing: 16.11.2011
(51) Int. Cl.: C07K 16/28

(54) **AGENTS AND METHODS FOR TREATING DISEASES THAT CORRELATE WITH BCMA EXPRESSION**

(30) Priority: 16.11.2010 EP 10191418
(62) Divisional of application: 11782175.1
(71) Applicant: Amgen Inc., Thousand Oaks CA 91320 (US); Amgen Research (Munich) GmbH, 81477 München (DE)
(72) Inventor: BORGES, Eric, 55216 Ingelheim am Rhein (DE); HEBEIS, Jasmin Barbara, 55216 Ingelheim am Rhein (DE)
(74) Representative: Dörries, Hans Ulrich

(57) **Abstract**

A bispecific binding agent comprising at least two binding domains, wherein a first binding domain binds to the B cell maturation antigen BCMA and wherein a second binding domain binds to CD3, pharmaceutical compositions containing such agent and use for the treatment of plasma cell disorders or B cell disorders which correlate with BCMA expression.

## Description

The present invention relates to agents and methods for the treatment of human diseases that correlate with BCMA expression, including tumor therapy, in particular the therapy of plasma cell disorders like multiple myeloma (MM), plasmacytoma and plasma cell leukemia and other B cell disorders like NHL, CLL HD, as well as autoimmune diseases.

To date, one of the most frequently used MM therapies includes chemotherapy. However, in most patients, chemotherapy is only able to partially control multiple myeloma, it rarely leads to complete remission. Agents used for treating the disease are Cyclophosphamid, Doxirubicin, Vincristin and Melphalan, combination therapies with immunomodulating agents such as thalidomide (Thalomid^{®}), lenalidomide (Revlimid^{®}), and bortezomib (Velcade^{®}) have emerged as important options for the treatment of myeloma, both in newly diagnosed patients and in patients with advanced disease in whom chemotherapy or transplantation have failed. In most cases, these agents are used in combination with standard chemotherapy agents. Other treatments use corticosteroids such as prednisone or dexamethasone. Another therapy is stem cell (bone marrow) transplantation. In multiple myeloma, most transplants are of the autologous type, i.e. using the patient's own cells. Such transplants, although not curative, have been shown to prolong life in selected patients. They can be performed as initial therapy in newly diagnosed patients or at the time of relapse. Sometimes, in selected patients, more than one transplant may be recommended to adequately control the disease.

The currently used therapies are usually not curative, most patients have to go through repeated treatment regimens during the course of their disease. Stem cell transplantation may not be an option for many patients because of advanced age, presence of other serious illness, or other physical limitations.

The relative survival rate measures the survival of multiple myeloma patients in comparison with the general population to estimate the effect of cancer. The overall five-year relative multiple myeloma survival rate for 1995-2001 was 32.4 percent.

It has been an object of the invention to provide novel agents and methods for the therapy of plasma cell disorders like MM.

The solution of the problem underlying the invention is based on the concept of generating a bispecific binding agent that contains one binding domain that is specific for CD3. According to this concept, the agent binds to T cells. The other binding domain is present due to its ability to pull the target cells, i.e. plasma cells, into the complex by specifically binding to a selective target molecule present on the target cells, thus making it possible to target the cytotoxic effect of the T cells to the plasma cells. When designed optimally, formation of this complex will induce signalling in cytotoxic T cells, either on its own or in combination with accessory cells, which leads to the release of cytotoxic mediators. If suitably designed, the agent will only induce the desired signalling in the presence of the target cell providing the surface molecules to form a signalling complex of CD3 molecules.

Hence, the present invention relates to a bispecific binding agent comprising at least two binding domains, comprising a first binding domain and a second binding domain, wherein said first binding domain binds to BCMA and wherein said second binding domain binds to CD3. (In the context of the invention, in the following, if not otherwise indicated, BCMA and CD3, which are the antigens for which the bispecific binding agents have binding specificity, are also termed "target molecules").

The first target molecule, BCMA (B cell maturation antigen; Accession No. AB052772, WO 00/40716), or tumor necrosis factor receptor superfamily member 17 (TNFRSF17), also known as CD269, is a protein that is encoded by the TNFRSF17 gene in humans (Treml et al., 2007, Semin. Immunol. 18 (5): 297-304; Mackay and Leung, 2007, Semin. Immunol. 18 (5): 284-9; Gras et al., 1996, Int. Immunol. 7 (7): 1093-106; Accession No. AB052772, WO 00/40716).

BCMA is a transmembrane protein that is preferentially expressed in mature B lymphocytes, i.e. plasma cells, and is considered to be important for B cell survival. It has been shown to specifically bind to the tumor necrosis factor (ligand) superfamily member 13b (TNFSF13B/TALL-1/BAFF), and to lead to NF-kappaB and MAPK8/JNK activation. BCMA also binds to various TRAP family members, and thus may transduce signals for cell survival and proliferation. BCMA expression is restricted to the B-cell lineage and mainly present on plasma cells and plasmablasts and to some extent on memory B cells, but virtually absent on peripheral and naive B cells. BCMA is also expressed on multiple myeloma (MM) cells, MM being a malignant B cell disorder with increased plasma cell numbers. Together with its family members transmembrane activator and cyclophylin ligand interactor (TACI) and B cell activation factor of TNF family (BAFF) receptor (BAFFR), BCMA regulates different aspects of humoral immunity, B-cell development and homeostasis. Expression of BCMA appears rather late in B-cell differentiation and contributes to the long term survival of plasmablasts and plasma cells in the bone marrow. In line with this finding, BCMA also supports growth and survival of MM cells.

Ryan et al. (Mol. Cancer Ther. 2007; 6(11) reported the generation of an antagonistic BCMA-specific antibody that prevents NF-κB activation which is associated with a potent pro-survival signaling pathway in normal and malignant B-cells. In addition, the antibody conferred potent antibody-dependent cell-mediated cytotoxicity (ADCC) to multiple myeloma cell lines in vitro which was significantly enhanced by Fc-engineering.

Bellucci et al. (Blood, 2005, 105(10) identified BCMA-specific antibodies in multiple myeloma patients after they had received donor lymphocyte infusions (DLI). Serum of these patients was capable of mediating BCMA-specific cell lysis by ADCC and CDC and was solely detected in patients with anti-tumor responses (4/9), but not in non-responding patients (0/6). The authors speculate that induction of BCMA-specific antibodies contributes to elimination of myeloma cells and long-term remission of patients.

BAFF and a proliferation inducing ligand (APRIL) are two ligands of the TNF super family that bind to and activate BCMA, TACI and BAFFR. By fusing the Fc-domain of human immunoglobulin to TACI, Zymogenetics, Inc. has generated Atacicept (TACI-Ig) to neutralize both these ligands and prevent receptor activation. Atacicept is currently in clinical trials for the treatment of Systemic Lupus Erythematosus (SLE, phase III), multiple sclerosis (MS, phase II) and rheumatoid arthritis (RA, phase II), as well as in phase I clinical trials for the treatment of the B-cell malignancies chronic lymphocytic leukaemia (CLL), non-Hodgkins lymphoma (NHL) and MM. In preclinical studies atacicept reduces growth and survival of primary MM cells and MM cell lines in vitro (Moreaux et al, Blood, 2004, 103) and *in vivo* (Yaccoby et al, Leukemia, 2008, 22, 406-13), demonstrating the relevance of TACI ligands for MM cells. Since most MM cells and derived cell lines express BCMA and TACI, both receptors might contribute to ligand-mediated growth and survival. These data suggest that antagonizing both BCMA and TACI might be beneficial in the treatment of plasma cell disorders. In addition, BCMA-specific antibodies that cross react with TACI have been described (WO 02/066516).

BCMA mRNA is highly elevated in the malignant plasma cell disorders MM, plasmacytoma and plasma cell leukemia. Expression in normal tissue is low and restricted to lymphoid tissues and colon. The nature of the cell types that express BCMA in colon still remains to be unravelled, since a very dense lymphoid network covers the digestive tract.

Preferably, the first binding domain of the bispecific binding agent binds to an epitope of BCMA that is located in the extracellular domain of the receptor protein (see e.g. WO 00/40716).

The second target molecule is CD3.

The CD3 complex denotes an antigen that is expressed on mature human T-cells, thymocytes and a subset of natural killer cells as part of the multimolecular T-cell receptor complex. The T cell receptor (TCR) complex comprises the TCRα and β chains as the central components, flanked by the CD3 complex consisting of a γ and a δ chain as well as two ε and ξ chains. CD3 is responsible for the signal transduction of the TCR. As described by Lin and Weiss, Journal of Cell Science 114, 243-244 (2001), activation of the TCR complex by binding of MHC-presented specific antigen epitopes results in the phosphorylation of immunoreceptor tyrosine-based activation motifs (ITAMs) by Src family kinases, triggering recruitment of further kinases which results in T cell activation including Ca2⁺ release. Clustering of CD3 on T cells, e.g. by immobilized anti-CD3-antibodies, leads to T cell activation similar to the engagement of the T cell receptor, but independent from its clone typical specificity. Due to its central role in modulating T cell activity, there have been attempts to develop molecules that are capable of binding TCR/CD3. Much of this work has focused on the generation of antibodies that are specific for the human CD3 antigen.

A representative of anti-CD3 antibodies is the murine monoclonal antibody OKT3, which was the first monoclonal antibody approved by the FDA. OKT3 has been reported to be a potent T cell mitogen (Van Wauve, J. Immunol. 124 (1980), 2708-18; US 4,361,549) and a potent T cell killer (Wong, Transplantation 50 (1990), 683-9). Other antibodies specific for the CD3 antigen have also been reported (WO 04/106380; US 20040202657; US 6,750,325; US 6,706,265; EP 0504350; and Clark et al., European J. Immunol. 1989, 19:381-388; US 5,968,509).

According to US 5, 885, 573, the murine antibody OKT3 was transferred into a human antibody framework in order to reduce its immunogenicity. Furthermore, US 5,885,573 discloses specific mutations in the Fc receptor ("FcR") -binding segment of OKT3 in the CH2 region that are expected to result in modified binding affinities for human FcR. US 5,929,212 discloses a recombinant antibody molecule in which the binding regions have been derived from the heavy and/or light chain variable regions of a murine anti-CD3 antibody, e.g. OKT3, and have been grafted into a human framework. WO 98/52975 discloses a mutated variant of the murine anti-CD3 antibody OKT3 which is considered to be more stable than the parental OKT3.

OKT3 has been used as potent immunosuppressive agent in clinical transplantation to treat allograft rejection. Several publications have described alterations such as humanization of OKT3 to reduce side effects associated with cytokine release due to cross-linking between T cells and FcyR-bearing cells and the human anti-mouse antibody (HAMA) response e.g. US 5,929,212; US 5,885,573). OKT3 or other anti-CD3-antibodies have also been described as immunopotentiating agents to stimulate T cell activation and proliferation (US 6,406,696; US 6,143,297; US 6,113,901; Yannelly 1990, J. Immunol. Meth. 1, 91-100). Anti-CD3-antibodies have also been described as agents used in combination with anti-CD28 antibodies to induce T cell proliferation (US 6,352,694). OKT3 has further been used by itself or as a component of a bispecific antibody to target cytotoxic T cells to tumor cells or virus-infected cells (Nitta 1990, Lancet 335, 368-376; Sanna 1995, Bio/Technology 13, 1221-1224; WO 99/54440).

The bispecific binding agent of the invention may contain, in addition to said first and second binding domain, a further binding domain to enhance selectivity for tumor cells or to increase affinity by bi-paratopic tumor cell binding. This can be achieved either by providing binding domains that bind to other antigens expressed on plasma cells, e.g. CS-1, HM1.24, CD38, CD138 or CD70 and by balancing affinities so that optimal affinity is achieved only after bispecific binding of plasma cells or tumor cells.

The bispecific binding agent may be in the format of an antibody molecule or of an antibody-like molecule or of a protein scaffold with antibody-like properties or of a cyclic peptide with at least two binding specificities.

Unless indicated or defined otherwise, all terms used have their usual meaning in the art, which will be clear to the skilled person. Reference is for example made to the standard handbooks, such as Sambrook et al, "Molecular Cloning: A Laboratory Manual" (2nd Ed.), VoIs. 1-3, Cold Spring Harbor Laboratory Press (1989); Lewin, "Genes IV", Oxford University Press, New York, (1990), and Roitt et al., "Immunology" (2nd Ed.), Gower Medical Publishing, London, New York (1989), as well as to the general background art cited herein. Furhermore, unless indicated otherwise, all methods, steps, techniques and manipulations that are not specifically described in detail can be performed and have been performed in a manner known *per se*, as will be clear to the skilled person. Reference is for example again made to the standard handbooks, to the general background art referred to above and to the further references cited therein;

Unless indicated otherwise, the terms "antibody" or "immunoglobulin" are used as general terms to include both the full-size antibody, the individual chains thereof, as well as all parts, domains or fragments thereof.

The term "antibody molecule" (or immunoglobulin or Ig) encompasses antibodies, in particular human antibodies, antibody fragments, antibody-like molecules and conjugates (e.g. with human serum albumin or in the form of immunoconjugates, e.g. with ¹³¹iodine, calicheamicin, auristatin or others) with any of the above mentioned antibody molecules. Antibodies include, but are not limited to, monoclonal, chimerized monoclonal, bi- or multispecifc antibodies. The term "antibody" shall encompass complete immunoglobulins comprising two heavy chains and two light chains, e.g. fully human antibodies as they are produced by lymphocytes and for example present in blood sera, monoclonal antibodies secreted by hybridoma cell lines, polypeptides produced by recombinant expression in host cells, which have the binding specificity of immunoglobulins or monoclonal antibodies, and molecules which have been derived from such immunoglobulins, monoclonal antibodies, or polypeptides by further processing or recombinant expression while retaining their binding specificity.

Antibody fragments or antibody-like molecules may contain only a portion of the constant region or lack the constant domain as long as they exhibit specific binding to the antigen. The choice of the type and length of the constant region depends, if no effector functions like complement fixation or antibody dependent cellular toxicity are desired, mainly on the desired pharmacological properties of the antibody protein. The antibody molecule will typically be a tetramer consisting of two light chain/heavy chain pairs, but may also be dimeric, i.e. consisting of a light chain/heavy chain pair, e.g. a Fab or Fv fragment, or it may be a monomeric single chain antibody (scFv). Antigen-binding antibody fragments or antibody-like molecules, including single-chain antibodies and linear antibodies, may comprise, on a single polypeptide, the variable region(s) alone or in combination with the entirety or a portion of the following: constant domain of the light chain, CH 1, hinge region, CH2, and CH3 domains, e.g. a so-called "SMIP^{®}" ("Small Modular Immunopharmaceutical"), which is an antibody-like molecule employing a single polypeptide chain as its binding domain Fv, which is linked to single-chain hinge and effector domains devoid of the constant domain CH1 (WO 02/056910). SMIP^{®}s can be prepared as monomers or dimers, but they do not assume the dimer-of-dimers structure of traditional antibodies. A so-called scorpion, an extension of a SMIP that has two binding specificities, is described in WO 2007/146968. VHHs or VHs, as defined below, also fall within the category of antibody-like molecules" or "antibody fragments".

The term "binding domain" or "or antigen-binding domain" refers to the regions within the bispecific binding agent that bind to/interact with the structure/antigen/epitope of the respective target molecule, i.e. BCMA or CD3, respectively. It can refer to the complete variable region or specifically to the complementarity determining regions (CDRs) that form the contact surface with the target molecule.

A bispecific binding agent of the invention (or each of its binding domains respectively) "binds to" or "specifically bind to", "has affinity for" and/or "has specificity for" or "interacts with" a structure/epitope/antigen with respect to its target molecules BCMA and CD3 or is "directed against" or is a "binding" molecule or has a "binding specificity" with respect to such structure/antigen/epitope with respect to its target molecules.

Generally, the term "specificity" refers to the number of different types of antigens or epitopes to which a particular antigen-binding molecule can bind. The specificity of an antigen-binding molecule can be determined based on its affinity and/or avidity (in this context, the term "antigen-binding molecule" refers to either the BCMA- or the CD3-binding domain, respectively.) The affinity, represented by the equilibrium constant for the dissociation of an antigen with an antigen-binding protein (KD), is a measure for the binding strength between an epitope and an antigen-binding site on the antigen-binding protein: the lesser the value of the KD, the stronger the binding strength between an epitope and the antigen-binding molecule (alternatively, the affinity can also be expressed as the affinity constant (KA), which is 1/KD). As will be clear to the skilled person, affinity can be determined in a manner known per se, depending on the specific antigen of interest. Avidity is the measure of the strength of binding between an antigen-binding molecule and the pertinent antigen. Avidity is related to both the affinity between an epitope and its antigen binding site on the antigen-binding molecule and the number of pertinent binding sites present on the antigen-binding molecule.

The terms "epitope" or "antigenic determinant", which may be used interchangeably, refer to the part of the target molecule that is recognized by the respective antigen-binding domain of the bispecific binding agent of the invention. Epitopes define the minimum binding site for an antibody or an antibody-like molecule thus convey specificity to said antigen-binding molecule

In a first aspect, the bispecific binding agent is in the format of a bispecific antibody molecule or a fragment thereof which has at least two binding domains, comprising a first binding domain and a second binding domain, wherein said first binding domain binds to BCMA and wherein said second binding domain binds to CD3.

Bispecific full-length antibodies may be obtained by covalently linking two monoclonal antibodies or by conventional hybrid-hybridoma techniques.

Covalent linking of two monoclonal antibodies is described in Anderson, Blood 80 (1992), 2826-34. In the context of this invention, one of the antibodies is specific for BCMA and the other one for CD3.By way of example, a BCMA specific antibody, e.g. Vicky-1 (Santa Cruz, # sc-57037) or Mab193 (R&D, #MAB193) is chemically linked to a monoclonal anti-CD3 antibody, e.g. OKT3 (ATCC CRL 8001) or another anti-CD3 antibody like WT32, anti-leu-4, UCHT-1, SPV-3TA or SPV-T3B.

Bispecific antibodies can also be obtained by the hybrid hybridoma technique. By way of example, a hybridoma that produces monoclonal anti-BCMA antibody, e.g. one obtained by immunization of mice or rat as known in the art, may be fused with a hybridoma that produces OKT3 (ATCC CRL 8001) or another anti-CD3 antibody like WT32, anti-leu-4, UCHT-1, SPV-3TA or SPV-T3B. The resulting fused cell line produces a number of different permutations of heavy and light chains of the different antibodies, and it is therefore necessary to purify the desired bispecific molecule, e.g. by affinity chromatography, using immobilized antigen as described in Anderson, Blood 80 (1992), 2826-34.

Because the yields from the hybrid hybridoma method are low, a preferred method for obtaining full Ig bispecific binding agents of the invention is based on the principle as described by Ridgway et al., Protein Engineering, vol. 9, no. 7, pp. 617-621, 1996. This method uses constant region frameworks that contain modifications in the CH3 domain that have been obtained by introducing a bulky amino acid into one and a smaller amino acid into the other chain, thereby creating a donor and an acceptor framework. The modification restrains pairing and only allows for heterodimer, but not for homodimer formation. In the context of this invention, the DNA molecules encoding the variable domains of an anti-BCMA antibody are cloned into a donor framework, and the DNA molecules encoding the variable domains of an anti-CD3 antibody, are cloned into an acceptor framework, or vice versa. The anti-BCMA antibody sequences can be derived from protein sequencing of the variable regions of the light and heavy chains of a BCMA-specific antibody, e.g. a commercially available one or an antibody obtained by methods as described herein, or they can be obtained by sequencing the RNA of a hybridoma generated by immunization with BCMA, using conventional methods, e.g. as described in Harlow and Lane (Antibodies, A Laboratory Manual (1988), Cold Spring Harbor). The anti-CD3 antibody sequences can be derived from published sequences, e.g. as disclosed in US 7,381,803 or in WO 2008/119567. The cDNAs encoding the components of the antibodies are then introduced into the same host cell, which then expresses two different scIg chains with two different specificities that will self assemble into heterodimers, thereby creating chimeric bi-specific antibodies containing an Fc portion.

In a preferred embodiment, the bispecific binding agent of the invention has, in addition to its function to bind to the target molecules BCMA and CD3, a third function. In this format, the bispecific binding agent is a trifunctional molecule by targeting plasma cells through binding to BCMA, mediating cytotoxic T cell activity through CD3 binding and providing a fully functional Fc constant domain mediating antibody-dependent cellular cytotoxicity through recruitment of effector cells like NK cells; examples of such trifunctional bispecific binding agent are agents termed Triomabs^{®}, as described in e.g. U S,655,1592, and also all antibody-like molecules with the same bispecific binding capacity, e.g. provided by a bispecific scFv fragment as described below coupled to a FcR binding domain.

Thus, according to this aspect, the bispecific binding agent of the invention has one binding domain for the interaction with BCMA, one binding domain for binding to CD3 and, as the third function, the intact Fc region with its functional binding features for activating Fcγ receptor type I (CD64)-, IIa (CD32a)- and III (CD16)- expressed on accessory cells. While, according to this aspect of the invention, the bispecific binding agent in the format of an intact bispecific antibody binds to the T cell with the binding arm that is specific for CD3 and activates it at the same time, co-stimulatory signals from the Fc receptor-positive cell bound to the Fc portion of the bispecific antibody can be transferred to the T cell.

Triomabs^{®} are produced by quadroma technology, as described e.g. in WO 95/33844, by fusion of a ratIgG2b BCMA-specific hybridoma with a mouseIgG2a CD3-specific hybridoma, thereby forming a quadroma. The obtained cells produce parental CD3 specific rat heavy and light chains and parental BCMA mouse heavy and light chains. These chains self-assemble to form homodimeric parental mouse and rat antibodies as well as chimeric antibodies of the two specificities. Chimeric antibodies, which are not humanized, have to be purified to get rid of the parental mono-specific antibodies. By way of example, the hybridoma that produces the CD3 specific antibody may be the mouse IgG2a producing OKT3 cell line, which is fused with a hybridoma, derived from immunization of rats as described, that produces a BCMA-specific ratIgG2b. The resulting bispecific binding agents are secreted into the supernatant by the quadroma cells and purified as described e.g. in WO 95/33844 using a two step process, wherein the first step is specific for the first species, said first step resulting in all homodimers of the first species and all heterodimeric molecules. The second purification process is specific for the second species and yields only heterodimeric molecules, producing purified trifunctional binding agents binding CD3 and BCMA as well as Fc receptors on effector cells.

In another embodiment, the bispecific binding agent is in the format of an antibody-like molecule with a heavy chain containing two consecutive N-terminal variable domains with different specificities and a light chain with two consecutive variable domains with different specificities resulting in four binding domains with two different specificities (Wu et al., Nat. Biotechnology, 2007, 25(11)), wherein one specificity is CD3 and the other specificity is BCMA.

In a further embodiment, the bispecific binding agent is in the format of an antibody fragment. An example of a bispecific antibody fragment is a diabody (Kipriyanov, Int. J. Cancer 77 (1998), 763-772), which is a small bivalent and bispecific antibody fragment. Diabodies comprise a heavy (VH) chain variable domain connected to a light chain variable domain (VL) on the same polypeptide chain (VH-VL) connected by a peptide linker that is too short to allow pairing between the two domains on the same chain. This forces pairing with the complementary domains of another chain and promotes the assembly of a dimeric molecule with two functional antigen binding sites.

To construct bispecific diabodies of the invention, the V-domains of an anti-CD3 antibody and an anti-BCMA antibody are fused to create the two chains VH(CD3)-VL(BCMA), VH(BCMA)-VL(CD3). Each chain by itself is not able to bind to the respective antigen, but recreates the functional antigen binding sites of anti-CD3 antibody and anti-BCMA antibody on pairing with the other chain. The two scFv molecules, with a linker between heavy chain variable domain and light chain variable domain that is too short for intramolecular dimerization, are co-expressed and self assemble to form bi-specific molecules with the two binding sites at opposite ends. By way of example, the variable regions encoding the binding domains for BCMA and CD3, respectively, can be amplified by PCR from DNA constructs obtained as described, such that they can be cloned into a vector like pHOG, as described in Kipiriyanov et al., J. Immunol. Methods, 200, 69-77 (1997a). The two scFV constructs are then combined in one expression vector in the desired orientation, whereby the VH-VL linker is shortened to prevent backfolding of the chains onto themselves. The DNA segments are separated by a STOP codon and a ribosome binding site (RBS). The RBS allows for the transcription of the mRNA as a bi-cistronic message, which is translated by ribosomes into two proteins which non-covalently interact to form the diabody molecule. Diabodies, like other antibody fragments, have the advantage that they can be expressed in bacteria (*E. coli*) and yeast (*Pichia pastoris*) in functional form and with high yields (up to 1g/l).

scFv molecules can be derived from antibodies or phage display as well known in the art, e.g. as described e.g. in Tungpradabkul et. al., Molecular Immunology, Volume 42, Issue 6, April 2005, 713-719; Yang D-F. et al., World J Gastroenterol 2005;11(21):3300-3303.

According to a preferred aspect, the bispecific binding agent of the invention is in the format of a bispecific single chain antibody construct, whereby said construct comprises or consists of at least two binding domains, whereby one of said domains binds to human BCMA and a second domain binds to human CD3. Such molecules, also termed "bispecific T cell engagers" (BiTEs^{®}) consist of two scFv molecules connected via a linker peptide; they have been described e.g. in WO 2004/106383, WO 2007/073499 and WO 2004/106381. A bispecific scFv is expressed as a fusion protein of the two heavy and two light chain variable regions in CHO cells. (The term BiTE^{®} only refers to bi-specific molecules of which one arm is specific for CD3). scFv are generally selected using a phage library generated from immunized animals, conversion of conventional rodent antibodies into scFv is also possible.

According to this aspect, the corresponding variable heavy chain regions (VH) and the corresponding variable light chain regions (VL) are arranged, from N-terminus to C-terminus, in the order
VH(BCMA)-VL(BCMA)-VH(CD3)-VL(CD3),
VH(CD3)-VL(CD3)-VH(BCMA)-VL(BCMA) or
VH CD3)-VL(CD3)-VL(BCMA)-VH(BCMA).

In a preferred embodiment of the invention, the VH and VL regions of the CD3-binding domain are derived, as suggested in WO 2004/106383, from an CD3 specific antibody selected from the group consisting of X35-3, VIT3, BMA030 (BW264/56), CLB-T3/3, CRIS7, YTH12.5, F111-409, CLB-T3.4. 2, TR-66, WT32, SPv-T3b, llD8, XIII-141, XIII-46, XIII-87, 12F6, T3/RW2-8C8, T3/RW2-4B6, OKT3D, M-T301, SMC2, WT31 and F101.01. These CD3-specific antibodies are well known in the art and have, inter alia, been described by Tunnacliffe (1989), Int. Immunol. 1, 546-550. According to a specific embodiment, said VH and VL regions of said CD3-specific domain are derived from the antibody OKT3 or a modified version thereof, as described above. In another embodiment, the VH and VL regions are those of or are derived from an antibody/antibody derivative with specificity for the CD3 molecule described by Traunecker (1991), EMBO J. 10, 3655-3659. Other examples of useful antibodies are described in WO 2008/119567 and in US 7,381,803.

Said VH and VL regions are derived from antibodies/antibody molecules and antibody-like molecules which are capable of specifically recognizing the human CD3 ε chain in the context of other TCR subunits as present on activated primary human T cells expressing the TCR in its native configuration. Accordingly, the VH and VL regions derived from an antibody specific for the CD3 ε chain are most preferred and said (parental) antibodies should be capable of specifically binding epitopes reflecting the native or near-native structure or a conformational epitope of human CD3 presented in the context of the TCR complex. As explained in WO 2004/106383, such antibodies have been classified as "group II" antibodies. Further classifications comprise the definition of "group I" and "group III" antibodies directed against CD3."Group I "antibodies, like UCHT1, recognize the CD3 ε chain expressed as recombinant protein and as part of the TCR on the cell surface. Therefore, "group I" antibodies are highly specific for the CD3 ε chain. In contrast, the "group II antibodies" recognize the CD3 ε chain only in the native TCR complex in association with other TCR subunits. Without wishing to be bound by theory, it may be speculated that in "group II" antibodies, the TCR context is required for recognition of CD3 ε chain.

The bispecific single chain antibody constructs described herein above and below may be humanized, as described in e.g. Gabbard et al., Methods for the humanization, Protein Engineering, Design & Selection vol. 22 no. 3 pp. 189-198, 2009 and/or deimmunized, as described in US 2009/0022738 of the bispecific antibody constructs of the invention are known to the person skilled in the art.

According to another embodiment, the two scFv molecules are linked, instead of by a linker (as in the BiTEs), by a human serum albumin (HSA) molecule, as described in WO 2009/126920, which serves to increase the half-life of the construct, which is expressed as a single molecule. Proteins in the plasma are constantly sampled by endothelial cells and usually degraded. However, the CH2-CH3 hinge region of antibodies can bind to the neonatal Fc receptor (FcRn) in acidified endosomes which will prevent degradation and lead to recycling of the antibody molecule. In addition to antibodies, FcRn is also able to bind HSA at a different site, which results in a similar recycling process that contributes to the extension of serum half-life of albumin and albumin bound molecules. In a variation of the teaching of WO 2009/126920, one binding domain is specific for BCMA and targets the molecule to the tumor cell, the other one binds to CD3 in view of CD3-mediated re-directed T cell lysis.

In yet another embodiment, the bispecific binding agent of the invention is in the format of a bispecific immunoglobulin single variable domain like a VHH and VH

The term "immunoglobulin single variable domain" as used herein means an immunoglobulin variable domain which is capable of specifically binding to an epitope of the antigen without pairing with an additional variable immunoglobulin domain. Examples of immunoglobulin single variable domains in the meaning of the present invention are the immunoglobulin single variable domains VH and VL and (VH domains and VL domains) and "VHH domains" (or simply "VHHs") from camelides, as defined hereinafter.

"VHH domains", also known as VHHs, V_{H}H domains, VHH antibody fragments, and VHH antibodies, have originally been described as the antigen binding immunoglobulin (variable) domain of "heavy chain antibodies" (i.e. of "antibodies devoid of light chains"; Hamers-Casterman C, Atarhouch T, Muyldermans S, Robinson G, Hamers C, Songa EB, Bendahman N, Hamers R.: "Naturally occurring antibodies devoid of light chains"; Nature 363, 446-448 (1993)). The term "VHH domain" has been chosen in order to distinguish these variable domains from the heavy chain variable domains that are present in conventional 4-chain antibodies ("VH domains") and from the light chain variable domains that are present in conventional 4-chain antibodies ("VL domains"). As opposed to VH or VL domains, which will normally not bind to an epitope as a single antigen binding domain, VHH domains can specifically bind to an epitope without an additional antigen binding domain. VHH domains are small, robust and efficient antigen recognition units formed by a single immunoglobulin domain.

In the context of the present invention, the terms VHH domain, VHH, V_{H}H domain, VHH antibody fragment, VHH antibody, as well as "Nanobody^{®}" and "Nanobody^{®} domain" ("Nanobody" being a trademark of the company Ablynx N.V.; Ghent; Belgium) are used interchangeably and are representatives of immunoglobulin single variable domains (having the general structure: FR1-CDR1-FR2-CDR2-FR3-CDR3-FR4 and specifically binding to an epitope without requiring the presence of a second immunoglobulin variable domain), and which are distingished from the VHs by the so-called "hallmark residues", as defined in e.g. WO 2009/109635, Fig. 1.

"VH domains" and "VL domains" (or simply "VHs" or VLs"), respectively, which are derived from 4-chain antibodies, in particular from human antibodies are "single domain antibodies", also known as "domain antibodies", "Dab"s, "Domain Antibodies", and "dAbs" (the terms "Domain Antibodies" and "dAbs" being used as trademarks by the GlaxoSmithKline group of companies) have been described in e.g. Ward, E.S., et al.: "Binding activities of a repertoire of single immunoglobulin variable domains secreted from Escherichia coli", Nature 341: 544-546 (1989); Holt, L.J. et al.: "Domain antibodies: proteins for therapy", TRENDS in Biotechnology 21(11): 484-490 (2003); and WO 2003/002609. Single domain antibodies correspond to the variable domains of either the heavy or light chains of non-camelid mammalian, in particular human antibodies. In order to bind an epitope as a single antigen binding domain, i.e. without being paired with a VL or VH domain, respectively, specific selection for such antigen binding properties is required, e.g. by using libraries of human single VH or VL domain sequences.

According to this aspect, the bispecific binding agents of the invention comprise one or more immunoglobulin single variable domains binding to the antigen BCMA and one or more immunoglobulin single variable domains binding to the antigen CD3. In a preferred aspect, the immunoglobulin single variable domains are VHHs.

More specifically, such bispecific binding agents of the invention essentially consist of or comprise (i) a first immunoglobulin single variable domain specifically binding to an epitope of BCMA and (ii) a second immunoglobulin single variable domain specifically binding to an epitope of CD3, wherein said immunoglobulin single variable domains are linked to each other in such a way that they may simultaneously bind to BCMA and CD3. A bispecific immunoglobulin single variable domain of the invention includes (at least) one anti-BCMA immunoglobulin single variable domain and (at least) one anti-CD3 immunoglobulin single variable domain. According to a specific embodiment of the invention, in case that a bispecific binding agent of the invention includes more than one anti-BCMA immunoglobulin single variable domain and/or more than one anti-CD3 immunoglobulin single variable domains, i.e. three, four or even more immunoglobulin single variable domains, at least two of the anti-BCMA immunoglobulin single variable domains or at least two of the anti-CD3 immunoglobulin single variable domains are directed against different epitopes within the BCMA or possibly the CD3 molecule, respectively. According to the invention, the two or more immunoglobulin single variable domains can be, independently of each other, VHHs or VHs, provided that these immunoglobulin single variable domains will bind the antigen, i.e. BCMA or CD3, respectively.

The two immunoglobulin single variable domains may be of the same type of immunoglobulin single variable domain, i.e. both immunoglobulin single variable domains are in the format of VHHs or VHs. Preferably, the first and the second immunoglobulin single variable domains are VHHs, preferably humanized VHHs. Accordingly, the invention relates to bispecific binding agents comprising an (optionally humanized) anti-BCMA VHH and an (optionally humanized) anti-CD3 VHH. However, the bispecific binding agents may as well be other bispecific immunoglobulin single variable domains, such as VHs.

The bispecific binding agent of the invention, when in the format of a bispecific immunoglobulin single variable domain, may be affinity-matured by having one or more alterations in one or more CDRs which result in an improved affinity for one or both of the target molecules, as compared to the respective parent target-binding molecule. Methods for affinity maturation are known in the art, for example, by Marks et al., 1992, Biotechnology 10:779-783; or Barbas, et al., 1994, Proc. Nat. Acad. Sci, USA 91: 3809-3813.; Shier et al., 1995, Gene 169:147-155; Yelton et al., 1995, Immunol. 155: 1994-2004; Jackson et al., 1995, J. Immunol. 154(7):3310-9; and Hawkins et al., 1992, J. Mol. Biol. 226(3): 889 896; Johnson KS and Hawkins RE, "Affinity maturation of antibodies using phage display", Oxford University Press 1996.

According to another aspect, the bispecific binding agent of the invention, e.g. when in the format of an immunoglobulin single variable domain, has been obtained by including a humanization step, i.e. by replacing one or more amino acid residues in the amino acid sequence of a naturally occurring VHH sequence by one or more of the amino acid residues that occur at the corresponding position(s) in a variable heavy domain of a conventional 4-chain antibody from a human being. This can be performed using methods known in the art, which can by routinely used by the skilled person. A humanized VHH domain may contain one or more fully human framework region sequences, and, in an even more specific embodiment, may contain human framework region sequences derived from the human germline Vh3 sequences DP-29, DP-47, DP-51, or parts thereof, or be highly homologous thereto. Thus, a humanization protocol may comprise the replacement of any of the VHH residues with the corresponding framework 1, 2 and 3 (FR1, FR2 and FR3) residues of germline VH genes such as DP 47, DP 29 and DP 51) either alone or in combination. Suitable framework regions (FR) of the immunoglobulin single variable domains of the invention can be selected from those as set out e.g. in WO 2006/004678 and specifically, include the so-called "KERE" and "GLEW" classes.

According to certain embodiments of the invention, the at least two immunoglobulin single variable domains can be connected with each other directly (i.e. without use of a linker) or via a linker. The linker is preferably a linker peptide and will be selected so as to allow binding of the at least two different immunoglobulin single variable domains to their respective target molecules. The linker comprised usually more than 17 amino acids, e.g. about 20 - 40 amino acid residues. The upper limit is not critical but is chosen for reasons of convenience regarding e.g. biopharmaceutical production of such polypeptides.

Also, the two or more immunoglobulin single variable domains may be linked to each other via a third VH or VHH, respectively (in such bispecific binding agents, the two or more immunoglobulin single variable domains may be linked directly to said third immunoglobulin single variable domain or via suitable linkers). Such a third VH or VHH may for example be a VH or VHH that provides for an increased half-life, as described below. For example, the latter VH or VHH may be a VH or VHH that is capable of binding to a (human) serum protein such as (human) serum albumin or (human) transferrin.

According to a preferred embodiment of the invention, the bispecific binding agent, when in the format of a bispecific immunoglobulin single variable domain, includes a moiety which extends the half-life of the polypeptide of the invention in serum or other body fluids of a patient, e.g. an Fc portion, an albumin moiety, a fragment of an albumin moiety, an albumin binding moiety, such as an anti-albumin immunoglobulin single variable domain, a transferrin binding moiety, such as an anti-transferrin immunoglobulin single variable domain, a polyoxyalkylene molecule, such as a polyethylene glycol molecule or an albumin binding peptide.

If the bispecific immunoglobulin single variable domain is modified by the attachment of a PEG moiety, the linker sequence preferably includes an amino acid residue, such as a cysteine or a lysine, allowing such modification, e.g. PEGylation, in the linker region. In another preferred embodiment, the polypeptide of the invention comprises a moiety which binds to an antigen found in blood, such as serum albumin.

In order to generate a bispecific binding agent of the present invention in the format of an immunoglobulin single variable domain, in a first step, a library of human VH or VL genes is screened for BCMA and CD3 specific binders e.g. by phage display, ribosome or RNA display. To this end, the extracellular domain of BCMA as a recombinant protein, e.g. as an Ig Fc fusion protein as described above, can be used in several display rounds to enrich for BCMA specific binders until highly specific phage binders are identified. The region encoding the CDRs can then be extracted by PCR amplification and cloned into a domain antibody format. In a similar procedure, CD3 specific CDRs are extracted, using, instead of recombinant protein e.g. a cell line that expresses the CD3 receptor complex to ensure that binding of the resulting agent is specific for the native conformation of CD3. To generate binders of the VHH type, a library e.g. phage display, is generated from B cells of Llamas that have been immunized with BCMA or CD3, respectively, as e.g. described in WO 2009/109572. In both cases, the resulting BCMA and CD3 specific scFv DNA molecules are cloned into a vector providing a linker to generate bispecific binding agent genes which can be expressed to generate the bispecific binding agents themselves by transformation of the vectors into a a bacterial host, e.g. *E. coli.*

According to another aspect, the bispecific binding agent of the invention is in the format of a single chain Fv molecule (i.e. a molecule formed by association of the immunoglobulin heavy and light chain variable domains, VH and VL, respectively) as described e.g. in WO 03/025018 and WO 03/048209. Such Fv molecules, which are known as TandAbs^{®} comprise four antibody variable domains, wherein (a) either the first two or the last two of the four variable domains bind intramolecularly to one another within the same chain by forming an antigen binding scFv in the orientation VH/VL or VL/VH (b) the other two domains bind intermolecularly with the corresponding VH or VL domains of another chain to form antigen binding VH/VL pairs. In a preferred embodiment, as suggested in WO 03/025018, the monomers of such Fv molecule comprise at least four variable domains of which two neighboring domains of one monomer form an antigen-binding VH-VL or VL-VH scFv unit. These two variable domains are linked by a peptide linker of at least 5 amino acid residues, which does not prevent the intramolecular formation of a scFv. At least two variable domains of the monomer are non-covalently bound to two variable domains of another monomer resulting in the formation of at least two additional antigen binding sites to form the multimerization motif; these two variable domains of each monomer are linked by a peptide linker of a maximum of 12 amino acids.

According to the present invention, such bispecific binding agent in the format of an Fv molecule that has four antibody variable domains has at least two specificities, whereby at least one binding domain is specific to human BCMA and at least one binding domain is specific to human CD3. These dimeric or multimeric Fv molecules can be prepared as described in WO 03/025018 and WO 03/048209. Methods of preparation are standard methods, e.g. ligating DNA sequences encoding the peptide linkers with the DNA sequences encoding the variable domains such that the peptide linkers connect the variable domains resulting in the formation of a DNA sequence encoding a monomer of the multimeric Fv-antibody and expressing DNA sequences encoding the various monomers in a suitable expression system.
By way of example, the variable regions of the VH and VL genes of a BCMA specific antibody and of a CD3 specific antibody are PCR-ampified using primers with restrictions sites allowing for the cloning the fragments into vectors in the orientation BCMA-VH CD3-VL x BCMA-VL CD3-VH, all separated by peptide linkers. These are then introduced into an expression system, prokaryotic or eukaryotic, and the resulting protein chains form homodimers resulting in two VH-VL associated binding domains for each specificity to generate a bispecific binding molecule for BCMA and CD3 binding.

In a further embodiment, the bispecific binding agent is in the format of a bispecific single-chain immunopharmaceutical. Such bispecific immunopharmaceutical, which is described in WO 2007/146968 and which was described as "SCORPION^{®}" due to the presence of so-called "scorpion linkers" in the molecule, is an extension of a so-called "SMIP"^{®}, an antibody-like molecule described above. The bispecific variant of such immunopharmaceutical is comprised of independent binding domains on each end of the molecule (BD1 and BD2), flanking an immunoglobulin constant domain region (e.g. IgG CH2 and CH3 domains) and may be produced by conventional methods in eukaroytic host cells, as described for SMIPs, e.g. in WO 02/056910.

As described in WO 2007/146968, a bispecific binding agent of the invention in the form of a single-chain binding scorpion molecule, consists of a first binding domain derived from an antibody or an antibody-like molecule specific for BCMA, a constant sub-region that is located C-terminal to the first binding domain and provides an effector function, a scorpion linker located C-terminal to the constant sub-region, and a second binding domain derived from an antibody or antibody-like molecule specific for CD3, which is located C-terminal to the constant sub-region. Thus, in such construct, the constant sub-region is localized between the first binding domain and the second binding domain. All of the domains of the protein are found in a single chain, but the protein may form homo-multimers, e.g., by interchain disulfide bond formation. By way of an example, the DNA molecules encoding the scFvs specific for BCMA or CD3 are generated, as described herein, by molecular cloning techniques known to the person skilled in the art. The CD3-specific VH and VL sequences are linked by a stretch of DNA encoding a peptide linker, thereby generating the CD3-binding domain that is inserted into a vector at the 5' end of a secretory leader sequence and at the 3' end of an immunoglobulin CH2-CH3 encoding sequence. The BCMA-specific single chain sequences, in the way as described for the CD3 sequences, are inserted at the 5' end of the CH2-CH3 sequence, separated from it by a stretch of DNA encoding a stretch of amino acids containing a cystein residue. This construct is transfected into a mammalian expression system like CHO cells. Upon cultivation, the expession product is found in the supernatant where it homodimerises via a disulfide bridge formed by said cystein residues to form a bispecific binding agent with bivalent binding sites for CD3 and BCMA as well as a functional Fc binding effector domain.

The bispecific binding agent of the invention may also be in the format of a synthetic immunoglobulin domain that has a binding domain engineered into a structural loop of an antibody or an antibody-like molecule, i.e. into a loop that is not a CDR loop and that does not contribute to antigen binding. This molecule is capable of binding to one antigen via its two conventional heavy chain/light chain Fv and to another antigen via its two additional binding sites that have been engineered into the structural loops, as described in e.g. WO 2006/072620, WO 2008/003103. By way of example, according to this embodiment, the bispecific binding agent may consist of a heavy chain and a light chain, which form together a variable region binding to a specific binding partner, i.e. BCMA, by a first specificity. The second specificity for CD3 is provided by a modified region containing a CD3-binding site in any of the structural loops of either the heavy chain or the light chain, e.g. within CH3. Such additional binding site may also be formed by more than one non-CDR loops which may be structurally neighboured (either on the heavy chain or on the light chain or on both chains). The binding site in the structural loop of the molecule may be derived from full IgG antibodies, antigen binding fragments (Fab), single chain antibodies (scFv) as well as diabodies or single domain antibodies, or selected from a display library as suggested in WO 2006/072620 or WO 2008/003103. By way of example, the CDRs of a BCMA-specific antibody derived from protein sequencing or from the RNA of a BCMA specific hybridoma, are cloned via standard molecular biology techniques into a full Ig framework as previously described to form a bivalent BCMA binding antibody. The CH3 of this recombinant antibody gene is modified by insertion of a CD3-binding domain derived from a known CD3 specific antibody, e.g. OKT3 or others described herein, by PCR amplification of the desired region with primers allowing for the insertion of the fragment via restriction enzyme treatment using standard molecular biology techniques and cloning into an expression vector. This results in an light chain DNA sequence encoding part of the BCMA-specific CDRs in its variable domain and a heavy chain DNA sequence encoding the other part of the BCMA-binding CDRs in its variable region, but also containing a CD3-specific binding domain in the CH3 constant region. When transfected and expressed e.g. in CHO cells, the resulting proteins expressed from the two DNA molecules will assemble to form homodimers of heavy and light chain heterodimers by disulfide bond formation containing bivalent binding sites for BCMA and CD3.

According to another embodiment, the bispecific binding agent is the format of a bispecific ankyrin repeat molecule as described e.g. in US 2009082274. These molecules (also known as "DARPins") are derived from natural ankyrin proteins, which can be found in the human genome and are one of the most abundant types of binding proteins. A DARPin library module is defined by natural ankyrin repeat protein sequences, using 229 ankyrin repeats for the initial design and another 2200 for subsequent refinement. The modules serve as building blocks for the DARPin libraries. The library modules resemble human genome sequences. A DARPin is composed of 4 to 6 modules. Because each module is ∼ 3.5 kDa, the size of an average DARPin is 16-21 kDa, which makes it similar to dAbs. This design makes them very stable proteins. Selection of binders is done by ribosome diplay, which is completely cell-free and is described in He M and Taussig MJ., Biochem Soc Trans. 2007, Nov;35(Pt 5):962-5. Ribosome display results in a complex of mRNA, ribosome, and protein which can bind to surface-bound ligand. This complex is then stabilized. During the subsequent binding, or panning, stages, the complex is introduced to surface-bound ligand. The complexes that bind well are immobilized and subsequently eluted to allow dissociation of the mRNA. The mRNA can then be reverse transcribed back into cDNA, undergo mutagenesis, and iteratively fed into the process with greater selective pressure to isolate even better binders.

In the context with the present invention, by way of example, two DARPins are selected using e.g. a BCMA-Fc fusion protein (R&D, #193-BC-050) as the immobilized surface-bound ligand for one and e.g. purified and mitogenically activated human T cells providing the CD3 receptor complex in its native form for the other ligand. After performance of ribosome display as described above, two independent darpin molecules of each specificity arise and can then be fused as suggested by Stumpp et al., Drug Discovery Today, Volume 13, Numbers 15/16, August 2008. The specificities and binding affinities of the resulting molecule can be analysed by flow cytometry using NCI H929 cells (ATCC CRL-9068) as BCMA expressing cells and the above mentioned activated primary human T cells as CD3 expressing cells.

In the bispecific binding agent of the invention, the two binding domains may be in identical or different formats as described above, e.g. the first binding domain may be an immunoglobulin single variable domain like a VHH or a VH and the second one a different immunoglobulin single variable domain or a BiTE or a diabody, respectively, or vice versa, the first binding domain may be a full-size antibody and the second one an antibody fragment like a diabody or vice versa, etc.

In a further embodiment, the bispecific binding agent is in the format of a combination of chemically constrained cyclic peptides. This technology (see e.g. WO 2008/013454) is based on repertoires of chemically constrained peptides which are subjected to Darwinian selection of peptides. In order to select for peptides binding to the desired antigen, they are displayed on the surface of bacteriophage which can be modified with an organochemical scaffold to create a diverse array of constrained peptides. Utilizing iterative selection, high affinity binding peptides can then be selected. Two variable regions flanked by cysteine residues are the basis of these libraries of peptides which are fused with the P3 phage coat protein. The fusion protein is displayed on the surface on the phage as two binding loops and subsequently modified with a halomethylarene. Selection takes place similarly to the process described for scFv or single domain antibodies by phage display using antigen coated to a surface, e.g. a plate or sepharose beads or using cells expressing the antigen. The process is repeated iteratively to enrich for highly specific binding and high affinities. The resulting peptides can be combined with others, if desired, and expressed in eukaryotic or prokaryotic cells. By way of example, recombinant BCMA-Fc or 6xHIS fusion protein is used as antigen for panning for cyclical peptide binding to BCMA in several rounds. This will enrich for and ultimately lead to the isolation of the BCMA-specific subunit. In a similar way, CD3 expressing cells, e.g. Jurkat are used to ensure binding of the resulting cyclical peptides to the native conformation of the CD3 complex. This will lead to isolation of the CD3 specific subunit. The DNA encoding for the identified peptides can then be cloned into a vector separated by a peptide linker resulting in a expression plasmid encoding for the bispecific binding agent. When transfected into a production cell line like CHO, or, suitably modified, transformed into *E. coli* cells or another expression system, the bispecific binding agent will be expressed and can be purified.

Preferably, the first binding domain of a bispecific binding agent of the invention binds to an epitope of BCMA that is located in the extracellular domain (as described in WO 00/40716).

With respect to BCMA, a bispecific binding agent of the invention binding to one species form of BCMA may cross-react with BCMA from one or more other species. For example, bispecific binding agents of the invention binding to human BCMA may exhibit cross-reactivity with BCMA from one or more other species of primates and/or with BCMA from one or more species of animals that are used in animal models for diseases, for example monkey (in particular Cynomolgus or Rhesus), mouse, rat, rabbit, pig, dog or) and in particular in animal models for plasma cell diseases. bispecific binding agents of the invention that show such cross-reactivity are advantageous in a research and/or drug development, since they allows the molecules of the invention to be tested in acknowledged disease models such as monkeys, in particular Cynomolgus or Rhesus, or mice and rats.

Therefore, in view of cross-reactivity with one or more target molecules from species other than human that is/are intended for use as an animal model during development of a therapeutic bispecific BCMA/CD3-binding agent, the binding domains of the bispecific binding agent recognize an epitope in a region of the target molecule that has a high degree of identity with the corresponding human molecule. By way of example, in view of using a mouse and/or a cynomolgus model, the BCMA binding domain of the bispecific binding agent of the invention recognizes, if the anti-BCMA antibody should cross-react both with cynomolgus and mouse (Accession No. NP_035738) BCMA, an epitope in the region spanning amino acids 11-19 or 23-29, or recognizes, if the antibody should cross react with cynomolgus BCMA, an epitope spanning the region of amino acids 1-19, 23-29 or 32-51 or conformational epitopes formed by the secondary and tertiary structure of the protein consisting of amino acids in the regions specified.

With respect to CD3, the bispecific binding agent of the invention preferably recognizes the ε chain of CD3 (GenBank Accession No.NM_000733), particular, it recognizes an epitope that corresponds to the first 27 N-terminal amino acids of CD3 epsilon or functional fragments of this 27 amino acid stretch, as disclosed in WO 2008/119567, i.e. an epitope that is part of an amino acid sequence comprised in the group consisting of SEQ ID NOs. 2, 4, 6, or 8 of the sequence listing disclosed in WO 2008/119567. Since it has not only been shown in WO 2008/119567 that interaction of binding molecules with this region does not lead to a change or modification of the conformation, sequence, or structure surrounding the antigenic determinant, but it was also shown that binding molecules recognizing said epitope in the N-terminal region are also cross-reactive with non-chimpanzee primate CD3 ε, said second binding domain of the bispecific binding agents of the invention preferably recognize this N-terminal epitope. The CD3-binding domain of the bispecific binding agents of the invention may thus be identical with or derived from the CD3-binding domains as defined in WO 2008/119567 by the sequences of the VH and VL chains.

The bispecific binding agents of the invention, when in the format of antibody-like molecules, can be generated based on the partial or complete sequences of anti-BCMA and anti-CD3 immunoglobulin molecules, respectively.

There are various recombinant methods available for generating CDR sequences with BCMA or CD3 specificity and for inserting them into the various scaffolds in order to obtain the desired bispecific binding agent format:
The CDRs (complementarity determining regions) of an antibody with BCMA specificity can be obtained by N-terminal sequencing, Edman degradation and mass spectrometry of a commercially available antibody, e.g. Vicky-1 (Santa Cruz, # sc-57037) or Mab193 (R&D, #MAB193), suitable techniques have been reviewed by Steen and Mann, Nature Reviews Molecular Cell Biology, 5:699-711, 2004. Once the framework has been identified and the sequences of the CDRs are known, the encoding DNA sequence is synthesized and grafted onto a framework with similar properties as compared to the parental one by molecular cloning methods as described in Gabbard et al., Protein Engineering, Design & Selection, vol. 22, no. 3, pp. 189-198, 2009. This framework can be part of a full IgG sequence to generate a bispecific full-sized antibody, a single chain Fv fragment to generate an antibody fragment-based molecule or part of the structural region of an antibody to provide an additional specificity. All the thus obtained molecules are tested for binding to BCMA using commercially available recombinant protein representing the extracellular domain of BCMA (R&D, #193-BC-050) in an ELISA assay or by flow cytometry using a cell line e.g. NCI H929 (ATCC, # ATCC CRL-9068) expressing BCMA, both methods being well known to the person skilled in the art.

Preferably, the BCMA-specific antibody is generated by immunization, e.g. by immunizing a mouse or a rat, as described in G. Kohler and C. Milstein (Nature 256 (1975), 495), in Harlow and Lane (Antibodies, A Laboratory Manual (1988), Cold Spring Harbor) or in Galfre (Meth. Enzymol. 73 (1981), 3). The commercially available extracellular domain of BCMA as a fusion protein with human IgG1 Fc (R&D, #193-BC-050) can be used as antigen, mixed with an adjuvant like ALUM or Freund's adjuvant injected intraperitoneally repeatedly to generate a BCMA-specific antibody titre. The titres can be measured in the murine serum using an ELISA assay on recombinant protein or a Flow Cytometry assay e.g. on the cell line described above. If a BCMA-specific signal is detectable, the spleen cells are fused with a mouse myeloma cell line as described in Harlow and Lane (Antibodies, A Laboratory Manual (1988), Cold Spring Harbor). Resulting supernatants are screened using the above-described assays to detect BCMA-specific antibodies. Positive clones are grown up to sufficient numbers and cells are lysed for the extraction of RNA, e.g using an RNAeasy kit available from Qiagen. The RNA is reverse-transcribed and a set of degenerated primers similar to the ones described in Dziegiel et al, Journal of Immunological Methods 182 (1995) 7-19, is used to amplify the variable regions of the antibody's heavy and light chains. Resulting DNA fragments are sequenced to confirm that they are heavy and light chains and then cloned into a vector fused e.g. to the human IgGl constant region of tool antibody genes as described in Orlandi et al. (Proc. Natl. Acad. Sci. USA, 1989, 86: 3833-3837). The resulting heavy and light chain genes are transfected in different combinations, if there are different sequences, into e.g. HEK 293 cells (ATCC, CRL-1573), e.g. by Amaxa Nucleofector^{®} (Lonza, Vervier, Belgium). These cells then express the antibody secreted into the supernatant, which can then be tested as described herein for BCMA specificity. In a similar way, DNA molecules encoding the variable regions of CD3-specific antibodies can be obtained and inserted into the desired frameworks; techniques to generate the antibodies by means of recombinant DNA technology are well known to the skilled artisan., e.g. as described by Kurucz et al., J. Immunol. 154 (1995), 4576; and Hollinger et al., Proc. Natl. Acad. Sci. USA 90 (1993), 6444; or in US 7,381,803. Alternatively, the DNA molecules encoding the variable regions or the CDRs respectively, can be synthesized based on published sequences of anti-CD3 antibodies. They may be derived from X35-3, VIT3, BMA030 (BW264/56), CLB-T3/3, CRIS7, YTH12.5, F111-409, CLB-T3.4.2, WT31, WT32, SPv-T3b, 11D8, XIII-141, XIII-46, XIII-87, 12F6, T3/RW2-8C8, T3/RW2-4B6, M-T301, SMC2 and F101.01. These CD3-specific antibodies are well known in the art and have e.g. been described in Tunnacliffe (1989), Int. Immunol. 1, 546-550, as mentioned above. The antibody VH and VL regions may also be derived from the anti-CD3 antibody OKT3 (US 4,361,549) or variants thereof, as e.g. described in US 5,885,573, US 5,885,573, US 5,929,212 or WO 98/52975 or from antibody TR-66.

Based on the obtained anti-BCMA and anti-CD3 immunoglobulin molecules, the respective variable regions or only CDR sequences can be used to be transferred into the desired bispecific binding agent formats as described herein.

The KD of the binding domain specific for BCMA of the bispecific binding agent is preferably in the range of 10⁻⁷- 5x10⁻⁹ M and the KD of the binding domain specific for CD3 is preferably in the range of 10⁻⁶ - 5x10⁻⁹ M. In a preferred embodiment, the KD value of the BCMA binding domain is lower than the KD value of the CD3 binding domain corresponding to a higher affinity of the BCMA binding domain compared to the CD3 binding domain.

The efficacy of a bispecific binding agent of the invention, and of compositions comprising the same, can be tested using any suitable in vitro assay, cell-based assay, in vivo assay and/or animal model known per se, or any combination thereof, depending on the specific disease or disorder of interest. Suitable assays and animal models are known to the skilled person, and for example include the assays described herein and in the Examples below.

Binding of the bispecific binding agent to BCMA can be tested using e.g. an enzyme-linked immunosorbent assay (ELISA), as it is well known to the person skilled in the art. By way of example, the antigen, e.g. in the form of BCMA-Fc, is coated to the plate and incubated with a dilution of the agents described above. The plate is then washed to remove unspecific binding and the bispecific binding agents are detected by a second or tertiary step, depending on the format of the agent. All formats of the bispecific binding agent that contain Fc can be detected with anti-Fc commercially avalable antibodies, preferably directly enzyme-conjugated, e.g. to horse radish peroxidase for chemiluminescent detection as known in the art. Formats containing Ig light chains can be detected using anti-kappa or anti-lambda antibodies according to the type of the light chain, non-antibody bispecific binding agents can be detected utilizing incorporated tags, e.g. HIS tags that can be detected using commercially available anti-HIS antibodies. For confirmation and measurement of CD3 binding, preferably a method is used that allows for presentation of the antigen in its native form, e.g. flow cytometry. In this method, cell lines like Jurkat that express the CD3 receptor complex, are incubated with the agent which is detected in a way similar to the detection by ELISA, except that secondary step reagents are used that are directly conjugated to fluorophores which can then be detected by a flow cytometer e.g. FACSCanto II by BD Biosciences , detecting the level of binding of the agent to antigen-positive cells.

The functional activity of the bispecific binding agent can be tested by flow cytometry based assays. By way of example, a cell line like CHO is transfected with a BCMA expression construct allowing for antigen expression on the cell surface. These cells are then incubated with the agent. Meanwhile, human CD8 positive T cells are purified from primary PBMCs (Peripheral Blood Mononuclear Cells), e.g. by MACS (Automated Magnetic Cell Sorting and Separation; Miltenyi), then these effector cells are added to the target cells that have been incubated with the bispecific binding agent for e.g. 24h. The cells are then stained with propidium idodide (PI) which only enters dead or dying cells and fluorescently labelled anti-CD8. Analysis by e.g. FACSCanto allows for the identification of dead non-T cells by their staining with PI and a ratio of dead against live cells can be calculated as a parameter for determining the efficiency of lysis.

The efficacy of a bispecific binding agent of the invention can also be tested in vivo, e.g. using a mouse model: In an example of such model, human MM xenograft cells, e.g. NCI H929 cells, are mixed with human T cells purified from primary PBMC and injected into a NOD/SCID mouse. Without treatment with an effective bispecific binding agent of interest, the xenograft will form measurable tumors. Treatment with effective agents after tunour injection prevents tumor growth which can be measured by caliper.

In another aspect, the invention relates to nucleic acid molecules that encode bispecific binding agents of the invention in the format of antibodies or antibody-like molecules or ankyrin repeat proteins or fragments or components thereof. Such nucleic acid molecules are also referred to herein as "nucleic acids of the invention" and may also be in the form of a genetic construct. A nucleic acid of the invention may be genomic DNA, cDNA or synthetic DNA (such as DNA with a codon usage that has been specifically adapted for expression in the intended host cell or host organism).

The nucleic acid of the invention may also be in the form of, may be present in and/or may be part of a vector, such as for example a plasmid, cosmid or YAC. The vector may especially be an expression vector, i.e. a vector that can provide for expression of the bispecific binding agent *in vitro* and/or *in vivo* (i.e. in a suitable host cell, host organism and/or expression system). Such expression vector generally comprises at least one nucleic acid of the invention that is operably linked to one or more suitable regulatory elements, such as promoter(s), enhancer(s), terminator(s), and the like. Such elements and their selection in view of expression of a specific sequence in a specific host are common knowledge of the skilled person.

The nucleic acids of the invention may be prepared or obtained in a manner known *per se* (e.g. by automated DNA synthesis and/or recombinant DNA technology), based on the information on the amino acid sequences for the polypeptides of the invention given herein, and/or can be isolated from a suitable natural source.

In another aspect, the invention relates to host cells that express or that are capable of expressing one or more bispecific binding agents of the invention; and/or that contain one or more a nucleic acids of the invention. According to a particularly preferred embodiment, said host cells are bacterial cells; other useful cells are yeast cells, fungal cells or mammalian cells.

Suitable bacterial cells include cells from gram-negative bacterial strains such as strains of *Escherichia coli*, *Proteus*, and *Pseudomonas*, and gram-positive bacterial strains such as strains of *Bacillus*, *Streptomyces*, *Staphylococcus*, and *Lactococcus*. Suitable fungal cell include cells from species of *Trichoderma*, *Neurospora*, and *Aspergillus*. Suitable yeast cells include cells from species of *Saccharomyces* (for example *Saccharomyces cerevisiae), Schizosaccharomyces* (for example *Schizosaccharomyces pombe*), *Pichia* (for example *Pichia pastoris* and *Pichia methanolica*), and *Hansenula*.

Suitable mammalian cells include for example CHO cells, BHK cells, HeLa cells, COS cells, 293 HEK and the like. However, amphibian cells, insect cells, plant cells, and any other cells used in the art for the expression of heterologous proteins can be used as well.

The invention further provides methods of manufacturing a bispecific binding agent of the invention, such methods generally comprising the steps of:
- culturing host cells comprising one or more nucleic acids encoding a bispecific binding agent or a fragment thereof, under conditions that allow expression of the bispecific binding agent or a fragment thereof, and optionally, in the case of distinct binding domain fragments, the assembly of such components, and
- recovering or isolating the bispecific binding agent expressed by the host cells from the culture; and
- optionally further purifying and/or modifying and/or formulating the bispecific binding agent of the invention.

For production on an industrial scale, preferred host organisms include strains of *E. coli*, *Pichiapastoris*, and *S. cerevisiae* that are suitable for large scale expression, production and fermentation, and in particular for large scale pharmaceutical expression, production and fermentation.

The choice of the specific expression system depends in part on the requirement for certain post-translational modifications, more specifically glycosylation. The production of a bispecific binding agent of the invention for which glycosylation is desired or required would necessitate the use of mammalian expression hosts that have the ability to glycosylate the expressed protein. In this respect, it will be clear to the skilled person that the glycosylation pattern obtained (i.e. the kind, number and position of residues attached) will depend on the cell or cell line that is used for the expression.

Bispecific binding agents of the invention produced in a cell as set out above can be produced either intracellullarly (e.g. in the cytosol, in the periplasma or in inclusion bodies) and then isolated from the host cells and optionally further purified; or they can be produced extracellularly (e.g. in the medium in which the host cells are cultured) and then isolated from the culture medium and optionally further purified.

Methods and reagents used for the recombinant production of polypeptides, such as specific suitable expression vectors, transformation or transfection methods, selection markers, methods of induction of protein expression, culture conditions, and the like, are known in the art. Similarly, protein isolation and purification techniques useful in a method of manufacture of a polypeptide of the invention are well known to the skilled person.

The present invention also relates to pharmaceutical compositions containing, as the active ingredient, at least one bispecific binding agent of the invention. For pharmaceutical use, a bispecific binding agent of the invention may be formulated as a pharmaceutical preparation or composition comprising at least one bispecific binding agent and at least one pharmaceutically acceptable carrier, diluent or excipient and/or adjuvant, and optionally one or more further pharmaceutically active polypeptides and/or compounds. By means of nonlimiting examples, such a formulation may be in a form suitable for oral administration or for parenteral administration (such as by intravenous, intramuscular or subcutaneous injection or intravenous infusion). Suitable administration forms - which may be solid, semi-solid or liquid, depending on the manner of administration, as well as methods and carriers for use in the preparation thereof, are well known to the skilled person.

Thus, in a further aspect, the invention relates to a pharmaceutical composition that contains at least one bispecific binding agent and at least one suitable carrier, diluent or excipient (i.e. suitable for pharmaceutical use), and optionally one or more further active substances. The bispecific binding agents of the invention may be formulated and administered in any suitable manner known per se: Reference is made to the standard handbooks, such as Remington's Pharmaceutical Sciences, 18th Ed., Mack Publishing Company, USA (1990), Remington, the Science and Practice of Pharmacy, 21th Edition, Lippincott Williams and Wilkins (2005); or the Handbook of Therapeutic Antibodies (S. Dubel, Ed.), Wiley, Weinheim, 2007 (see for example pages 252-255).

For example, an immunoglobulin single variable domain of the invention may be formulated and administered in any manner known per se for conventional antibodies, antibody-like molecules and antibody fragments (including, but not limited to ScFv's and diabodies) and other pharmaceutically active proteins. Such formulations and methods for preparing the same are known to the skilled person.

Preparations for parenteral administration may for example be sterile solutions, suspensions, dispersions or emulsions that are suitable for infusion or injection. Suitable carriers or diluents for such preparations for example include, without limitation, sterile water and pharmaceutically acceptable aqueous buffers and solutions such as physiological phosphate-buffered saline, Ringer's solutions, dextrose solution, and Hank's solution; water oils; glycerol; ethanol; glycols such as propylene glycol or as well as mineral oils, animal oils and vegetable oils, for example peanut oil, soybean oil, as well as suitable mixtures thereof. Usually, aqueous solutions or suspensions will be preferred.

Thus, the bispecific binding agent of the invention may be systemically administered, e.g., orally, in combination with a pharmaceutically acceptable vehicle such as an inert diluent or an assimilable edible carrier. For oral therapeutic administration, the bispecific binding agent of the invention may be combined with one or more excipients and used in the form of ingestible tablets, buccal tablets, troches, capsules, elixirs, suspensions, syrups, wafers, and the like. Such compositions and preparations should contain at least 0.1% of the bispecific binding agent of the invention. Their percentage in the compositions and preparations may, of course, be varied and may conveniently be between about 2 to about 60% of the weight of a given unit dosage form. The amount of the bispecific binding agent of the invention in such therapeutically useful compositions is such that an effective dosage level will be obtained. The tablets, pills, capsules, and the like may also contain binders, excipients, disintegrating agents, lubricants and sweetening or flavouring agents. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac or sugar and the like. A syrup or elixir may contain the bispecific binding agents of the invention, sucrose or fructose as a sweetening agent, methyl and propylparabens as preservatives, a dye and flavoring such as cherry or orange flavor. Of course, any material used in preparing any unit dosage form should be pharmaceutically acceptable and substantially non-toxic in the amounts employed. In addition, the bispecific binding agents of the invention may be incorporated into sustained-release preparations and devices.

Preparations and formulations for oral administration may also be provided with an enteric coating that will allow the constructs of the invention to resist the gastric environment and pass into the intestines. More generally, preparations and formulations for oral administration may be suitably formulated for delivery into any desired part of the gastrointestinal tract. In addition, suitable suppositories may be used for delivery into the gastrointestinal tract.

The bispecific binding agents of the invention may also be administered intravenously or intraperitoneally by infusion or injection.

The amount of the bispecific binding agents of the invention required for use in treatment will vary not only with the particular bispecific binding agent selected, but also with the route of administration, the nature of the condition being treated and the age and condition of the patient and will be ultimately at the discretion of the attendant physician or clinician. The desired dose may conveniently be presented in a single dose or as divided doses administered at appropriate intervals, for example, as two, three, four or more sub-doses per day. The sub-dose itself may be further divided, e.g., into a number of discrete loosely spaced administrations; such as multiple inhalations from an insufflator or by application of a plurality of drops into the eye.

An administration regimen may include long-term, daily treatment. By "long-term" is meant at least two weeks and preferably, several weeks, months, or years of duration. Necessary modifications in this dosage range may be determined by one of ordinary skill in the art using only routine experimentation given the teachings herein. See Remington's Pharmaceutical Sciences (Martin, E.W., ed. 4), Mack Publishing Co., Easton, PA. The dosage can also be adjusted by the individual physician in the event of any complication.

According to a further embodiment, the invention relates to the use of bispecific binding agents of the invention for therapeutic purposes, such as
- for the prevention, treatment and/or alleviation of a plasma cell disorder or disease or a B cell disorder which correlates with BCMA expression, especially in a human being,
- in a method of treatment of a patient in need of such therapy, such method comprising administering, to a subject in need thereof, a pharmaceutically active amount of at least one bispecific binding agent of the invention, or a pharmaceutical composition containing same;
- for the preparation of a medicament for the prevention, treatment or alleviation of prevention, treatment and/or alleviation of a plasma cell disorder or disease;
- as an active ingredient in a pharmaceutical composition or medicament used for the above purposes.

According to a specific aspect, said disorder, disease or condition is a cancerous disease, in particular a plasma cell disorder or a B cell disorder which correlates with enhanced BCMA expression, as defined herein.

Plasma cell disorders include plasmacytoma, plasma cell leukemia, multiple myeloma, macroglobulinemia, amyloidosis, Waldenstrom's macroglobulinemia, solitary bone plasmacytoma, extramedullary plasmacytoma, osteosclerotic myeloma (POEMS Syndrome) and heavy chain diseases as well as the clinically unclear monoclonal gammopathy of undetermined significance/smoldering multiple myeloma.

Examples for B cell disorders which correlate with elevanted BCMA expression levels are CLL (chronic lymphocytic leukemia) and non-Hodgkins lymphoma (NHL). The bispecific binding agents of the invention may also be used in the therapy of autoimmune diseases like Systemic Lupus Erythematosus (SLE), multiple sclerosis (MS) and rheumatoid arthritis (RA).

Depending on the disease to be treated, a bispecific binding agent of the invention may be used on its own or in combination with one or more additional therapeutic agents, in particular selected from chemotherapeutic agents like DNA damaging agents or therapeutically active compounds that inhibit signal transduction pathways or mitotic checkpoints in cancer cells.

The additional therapeutic agent may be administered simultaneously with, optionally as a component of the same pharmaceutical preparation, or before or after administration of the bispecific binding agent of the invention.

In certain embodiments, the additional therapeutic agent may be, without limitation, one or more inhibitors selected from the group of inhibitors of EGFR, VEGFR, HER2-neu, Her3, Aurora A, Aurora B, PLK and PI3 kinase, FGFR, PDGFR, Raf, KSP, PDK1, PTK2, IGF-R or IR.

Further examples of additional therapeutic agents are inhibitors of CDK, Akt, src/bcr abl, cKit, cMet/HGF, c-Myc, Flt3, HSP90, hedgehog antagonists, inhibitors of JAK/STAT, Mek, mTor, NFkappaB, the proteasome, Rho, an inhibitor of wnt signaling or an inhibitor of the ubiquitination pathway or another inhibitor of the Notch signaling pathway.

Examples for Aurora inhibitors are, without limitation, PHA-739358, AZD-1152, AT 9283, CYC-116, R-763, VX-680, VX-667, MLN-8045, PF-3814735.

An example for a PLK inhibitor is GSK-461364.

Examples for raf inhibitors are BAY-73-4506 (also a VEGFR inhibitor), PLX 4032, RAF-265 (also in addition a VEGFR inhibitor), sorafenib (also in addition a VEGFR inhibitor), and XL 281.

Examples for KSP inhibitors are ispinesib, ARRY-520, AZD-4877, CK-1122697, GSK 246053A, GSK-923295, MK-0731, and SB-743921.

Examples for a src and/or bcr-abl inhibitors are dasatinib, AZD-0530, bosutinib, XL 228 (also an IGF-1R inhibitor), nilotinib (also a PDGFR and cKit inhibitor), imatinib (also a cKit inhibitor), and NS-187.

An example for a PDK1 inhibitor is BX-517.

An example for a Rho inhibitor is BA-210.

Examples for PI3 kinase inhibitors are PX-866, BEZ-235 (also an mTor inhibitor), XL 418 (also an Akt inhibitor), XL-147, and XL 765 (also an mTor inhibitor).

Examples for inhibitors of cMet or HGF are XL-184 (also an inhibitor of VEGFR, cKit, Flt3), PF-2341066, MK-2461, XL-880 (also an inhibitor of VEGFR), MGCD-265 (also an inhibitor of VEGFR, Ron, Tie2), SU-11274, PHA-665752, AMG-102, and AV-299.

An example for a c-Myc inhibitor is CX-3543.

Examples for Flt3 inhibitors are AC-220 (also an inhibitor of cKit and PDGFR), KW 2449, lestaurtinib (also an inhibitor of VEGFR, PDGFR, PKC), TG-101348 (also an inhibitor of JAK2), XL-999 (also an inhibitor of cKit, FGFR, PDGFR and VEGFR), sunitinib (also an inhibitor of PDGFR, VEGFR and cKit), and tandutinib (also an inhibitor of PDGFR, and cKit).

Examples for HSP90 inhibitors are tanespimycin, alvespimycin, IPI-504 and CNF 2024.

Examples for JAK/STAT inhibitors are CYT-997 (also interacting with tubulin), TG 101348 (also an inhibitor of Flt3), and XL-019.

Examples for Mek inhibitors are ARRY-142886, PD-325901, AZD-8330, and XL 518.

Examples for mTor inhibitors are temsirolimus, AP-23573 (which also acts as a VEGF inhibitor), everolimus (a VEGF inhibitor in addition). XL-765 (also a PI3 kinase inhibitor), and BEZ-235 (also a PI3 kinase inhibitor).

Examples for Akt inhibitors are perifosine, GSK-690693, RX-0201, and triciribine.

Examples for cKit inhibitors are AB-1010, OSI-930 (also acts as a VEGFR inhibitor), AC-220 (also an inhibitor of Flt3 and PDGFR), tandutinib (also an inhibitor of Flt3 and PDGFR), axitinib (also an inhibitor of VEGFR and PDGFR), XL-999 (also an inhibitor of Flt3, PDGFR, VEGFR, FGFR), sunitinib (also an inhibitor of Flt3, PDGFR, VEGFR), and XL-820 (also acts as a VEGFR- and PDGFR inhibitor), imatinib (also a bcr-abl inhibitor), nilotinib (also an inhibitor of bcr-abl and PDGFR).

Examples for hedgehog antagonists are IPI-609 and CUR-61414.

Examples for CDK inhibitors are seliciclib, AT-7519, P-276, ZK-CDK (also inhibiting VEGFR2 and PDGFR), PD-332991, R-547, SNS-032, PHA-690509, and AG 024322.

Examples for proteasome inhibitors are bortezomib, carfilzomib, and NPI-0052 (also an inhibitor of NFkappaB).

An example for an NFkappaB pathway inhibitor is NPI-0052.

An example for an ubiquitination pathway inhibitor is HBX-41108.

The additional therapeutic agent may also be an anti-angiogenic agent.

Examples for anti-angiogenic agents are inhibitors of the FGFR, PDGFR and VEGFR or the respective ligands (e.g VEGF inhibitors like pegaptanib or the anti-VEGF antibody bevacizumab), and thalidomides, such agents being selected from, without limitation, bevacizumab, motesanib, CDP-791, SU-14813, telatinib, KRN-951, ZK-CDK (also an inhibitor of CDK), ABT-869, BMS-690514, RAF-265, IMC-KDR, IMC-18F1, IMiDs (immunomodulatory drugs), thalidomide derivative CC-4047, lenalidomide, ENMD 0995, IMC-D11, Ki 23057, brivanib, cediranib, XL-999 (also an inhibitor of cKit and Flt3), 1B3, CP 868596, IMC 3G3, R-1530 (also an inhibitor of Flt3), sunitinib (also an inhibitor of cKit and Flt3), axitinib (also an inhibitor of cKit), lestaurtinib (also an inhibitor of Flt3 and PKC), vatalanib, tandutinib (also an inhibitor of Flt3 and cKit), pazopanib, GW 786034, PF-337210, IMC-1121B, AVE-0005, AG-13736, E-7080, CHIR 258, sorafenib tosylate (also an inhibitor of Raf), RAF-265 (also an inhibitor of Raf), vandetanib, CP-547632, OSI-930, AEE-788 (also an inhibitor of EGFR and Her2), BAY-57-9352 (also an inhibitor of Raf), BAY-73-4506 (also an inhibitor of Raf), XL 880 (also an inhibitor of cMet), XL-647 (also an inhibitor of EGFR and EphB4), XL 820 (also an inhibitor of cKit), and nilotinib (also an inhibitor of cKit and brc-abl).

The additional therapeutic agent may also be selected from EGFR inhibitors, it may be a small molecule EGFR inhibitor or an anti-EGFR antibody. Examples for anti-EGFR antibodies, without limitation, are cetuximab, panitumumab, matuzumab; an example for a small molecule EGFR inhibitor is gefitinib. Another example for an EGFR modulator is the EGF fusion toxin.

Among the EGFR and Her2 inhibitors useful for combination with the bispecific binding agent of the invention are lapatinib, gefitinib, erlotinib, cetuximab, trastuzumab, nimotuzumab, zalutumumab, vandetanib (also an inhibitor of VEGFR), pertuzumab, XL-647, HKI-272, BMS-599626 ARRY-334543, AV 412, mAB-806, BMS-690514, JNJ-26483327, AEE-788 (also an inhibitor of VEGFR), ARRY-333786, IMC-11F8, Zemab.

The additional agent may also be an IL-6 or an IL-6 receptor antagonist, e.g. atlizumab (tocilizumab).

Other agents that may be advantageously combined in a therapy with the bispecific binding agent of the invention are tositumumab and ibritumomab tiuxetan (two radiolabelled anti-CD20 antibodies), alemtuzumab (an anti-CD52 antibody), denosumab (an osteoclast differentiation factor ligand inhibitor), galiximab (a CD80 antagonist), ofatumumab (a CD20 inhibitor), zanolimumab (a CD4 antagonist), SGN40 (a CD40 ligand receptor modulator), rituximab (a CD20 inhibitor) or mapatumumab (a TRAIL-1 receptor agonist).

Other chemotherapeutic drugs that may be used in combination with the bispecific binding agents of the present invention are selected from, but not limited to hormones, hormonal analogues and antihormonals (e.g. tamoxifen, toremifene, raloxifene, fulvestrant, megestrol acetate, flutamide, nilutamide, bicalutamide, cyproterone acetate, finasteride, buserelin acetate, fludrocortisone, fluoxymesterone, medroxyprogesterone, octreotide, arzoxifene, pasireotide, vapreotide), aromatase inhibitors (e.g. anastrozole, letrozole, liarozole, exemestane, atamestane, formestane), LHRH agonists and antagonists (e.g. goserelin acetate, leuprolide, abarelix, cetrorelix, deslorelin, histrelin, triptorelin), antimetabolites (e.g. antifolates like methotrexate, pemetrexed, pyrimidine analogues like 5 fluorouracil, capecitabine, decitabine, nelarabine, and gemcitabine, purine and adenosine analogues such as mercaptopurine thioguanine, cladribine and pentostatin, cytarabine, fludarabine); antitumor antibiotics (e.g. anthracyclines like doxorubicin, daunorubicin, epirubicin and idarubicin, mitomycin-C, bleomycin dactinomycin, plicamycin, mitoxantrone, pixantrone, streptozocin); platinum derivatives (e.g. cisplatin, oxaliplatin, carboplatin, lobaplatin, satraplatin); alkylating agents (e.g. estramustine, meclorethamine, melphalan, chlorambucil, busulphan, dacarbazine, cyclophosphamide, ifosfamide, hydroxyurea, temozolomide, nitrosoureas such as carmustine and lomustine, thiotepa); antimitotic agents (e.g. vinca alkaloids like vinblastine, vindesine, vinorelbine, vinflunine and vincristine; and taxanes like paclitaxel, docetaxel and their formulations, larotaxel; simotaxel, and epothilones like ixabepilone, patupilone, ZK-EPO); topoisomerase inhibitors (e.g. epipodophyllotoxins like etoposide and etopophos, teniposide, amsacrine, topotecan, irinotecan) and miscellaneous chemotherapeutics such as amifostine, anagrelide, interferone alpha, procarbazine, mitotane, and porfimer, bexarotene, celecoxib.

### Example

### Generating a bispecific BCMA/CD3 single chain binding agent

### a) Generation of anti-BCMA and anti-CD3 binding domains

The DNA fragment encoding the CD3-specific binding domain is obtained by amplification from a synthetic DNA construct encoding the VH and VL region separated by an 18 amino acid linker, as disclosed in WO 2004106383, using primers similar to the ones described there, generating a BsrGl restriction site at the VH end and a BspEl restriction site at the VL end. The DNA sequence encoding the BCMA-binding domain is obtained by amplification from VH and VL DNA molecules synthesized upon sequencing a commercially available antibody. Alternatively, cDNA constructs are used that are obtained from the VH and VL RNA from a BCMA specific hybridoma, using suitable primers generating a BspEl restriction site at the VL 5' end and a SalI restriction site at the 3' VH end.

### b) Cloning of anti-CD3 x anti-BCMA constructs

Cloning is done in VH anti-CD3-VL anti CD3 x VH anti-BCMA-VL anti-BCMA orientation. The anti-CD3 construct is cleaved with the restriction enzymes BsrG1 and BspEl and subsequently cloned into the bluescript KS vector (Stratagene, La Jolla, CA), containing the amino acid sequence of an eukaryotic secretory signal (leader) peptide as a EcoRl/BsrGI-fragment. After cleavage with EcoRl and BspEl, the resulting DNA fragment comprising the respective anti-CD3 scFv with the leader peptide is cloned into an EcoRl/BspEl-cleaved plasmid pEFDHFR and the BCMA fragments are cloned into the BspE1/SalI-cleaved vector. Alternatively, cloning is done in the other orientation.

### c) Expression and characterization of the bispecific single chain binding agent

After confirmation of the desired sequence by DNA sequencing, the construct obtained in b) is transfected, e.g. into dehydrofolate reductase negative CHO cells, and expressed for characterisation as described in WO 2004/106383. For example, for binding to Jurkat cells (ATCC, # TIB-152) for CD3 and NCI H929 (ATCC CRL-9068) for BCMA a flow cytometry experiment is performed. The cells are incubated with the supernatant of BCMA/CD3 bi-specific construct expressing cells for approximately 1 h at 4°C, washed 2x in FACS buffer (phosphate-buffered saline containing 1 % fetal calf serum (FCS) and 0.05% sodium azide) and bound construct is detected via the 6xHIS tag incorporated in the expression vector pEFDHFR using a HIS antibody e.g. (Dianova, DIA910). For the detection of bound anti-HIS antibody the cells are washed as described above and incubated with e.g. goat anti-mouse-FITC-conjugated antibody (BD 550003) or with anti-mouse-PE conjugated antibody (IgG) (Sigma, P8547) and analysed e.g. on a FACS Canto (BD). The functional activity of the constructs is then analysed using a flow cytometry based assay after the constructs have been purified by a two-step purification process including immobilized metal affinity chromatography (IMAC) and gel filtration as described in WO 2004/106383, but using a CHO cell line transfected with a DNA construct expressing full-length BCMA on the surface.

In view of the above, it will be appreciated that the invention also encompasses the following items:
1. A bispecific binding agent comprising at least two binding domains, comprising a first binding domain and a second binding domain, wherein said first binding domain binds to the B cell maturation antigen BCMA and wherein said second binding domain binds to CD3.
2. The bispecific binding agent of item 1, wherein said first binding domain binds to the extracellular domain of BCMA and said second binding domain binds to the ε chain of CD3 .
3. A bispecific binding agent of items 1 or 2 which is in the format of a full-length antibody or an antibody fragment.
4. A bispecific binding agent of item 3 in the format of a full-length antibody, wherein said first BCMA-binding domain is derived from mouse said and wherein said second CD3-binding domain is derived from rat.
5. A bispecific binding agent of item 3, which is in the format of an antibody fragment in the form of a diabody that comprises a heavy chain variable domain connected to a light chain variable domain on the same polypeptide chain such that the two domains do not pair.
6. A bispecific binding agent of item 1 or 2 which is in the format of a bispecific single chain antibody that consists of two scFv molecules connected via a linker peptide or by a human serum albumin molecule.
7. The bispecific binding agent of item 6, heavy chain regions (VH) and the corresponding variable light chain regions (VL) are arranged, from N-terminus to C-terminus, in the order
   VH(BCMA)-VL(BCMA)-VH(CD3)-VL(CD3),
   VH(CD3)-VL(CD3)-VH(BCMA)-VL(BCMA) or
   VH CD3)-VL(CD3)-VL(BCMA)-VH(BCMA).
8. A bispecific binding agent of items 1 or 2, which is in the format of a single domain immunoglobulin domain selected from VHHs or VHs.
9. The bispecific binding agent of items 1 or 2, which is in the format of an Fv molecule that has four antibody variable domains with at least two binding domains, wherein at least one binding domain is specific to human BCMA and at least one binding domain is specific to human CD3.
10. A bispecific binding agent of items 1 or 2, which is in the format of a single-chain binding molecule consisting of a first binding domain specific for BCMA, a constant sub-region that is located C-terminal to said first binding domain, a scorpion linker located C-terminal to the constant sub-region, and a second binding domain specific for CD3, which is located C-terminal to said constant sub-region.
11. The bispecific binding agent of items 1 or 2, which is in the format of an antibody-like molecule that binds to BCMA via the two heavy chain/light chain Fv of an antibody or an antibody fragment and which binds to CD3 via a binding domain that has been engineered into non-CDR loops of the heavy chain or the light chain of said antibody or antibody fragment.
12. A bispecific binding agent of item 1 which is in the format of a bispecific ankyrin repeat molecule.
13. A bispecific binding agent of item 1, wherein said first binding domain has a format selected from the formats defined in any one of items 3 to 12 and wherein said second binding domain has a different format selected from the formats defined in any one of items 3 to 12.
14. A bispecific binding agent of item 1 which is a bicyclic peptide.
15. A pharmaceutical composition containing at least one bispecific binding agent of any one of items 1 to 14.
16. A bispecific binding agent of any one of items 1 to 14 or a pharmaceutical composition of item 14 for the treatment of plasma cell disorders or other B cell disorders that correlate with BCMA expression and for the treatment of autoimmune diseases.
17. A bispecific binding agent of any one of items 1 to 14 or a pharmaceutical composition of item 15 for the treatment of plasma cell disorders selected from plasmacytoma, plasma cell leukemia, multiple myeloma, macroglobulinemia, amyloidosis, Waldenstrom's macroglobulinemia , solitary bone plasmacytoma, extramedullary plasmacytoma, osteosclerotic myeloma, heavy chain diseases, monoclonal gammopathy of undetermined significance, smoldering multiple myeloma.

## Claims

1. A bispecific binding agent comprising at least two binding domains, comprising a first binding domain and a second binding domain, wherein said first binding domain binds to the B cell maturation antigen BCMA and wherein said second binding domain binds to CD3.

2. The bispecific binding agent of claim 1, wherein said first binding domain binds to an epitope of BCMA that is located in the extracellular domain.

3. The bispecific binding agent of claim 1 or 2, which recognizes the ε chain of CD3 (GenBank Accession No. NM 000733).

4. The bispecific binding agent of claim 3, which recognizes an epitope that corresponds to the first 27 N-terminal amino acids of CD3 epsilon or functional fragments of this 27 amino acid stretch.

5. The bispecific binding agent of any one of the preceding claims, which is in the format of a full-length antibody, an antibody fragment, an antibody molecule or an antibody-like molecule.

6. The bispecific binding agent of claim 5, wherein the antibody fragment or the antibody-like molecule contains only a portion of a constant region or lacks a constant domain.

7. The bispecific binding agent of any one of the preceding claims, which is in the format of a bispecific single chain antibody construct, whereby said construct comprises or consists of at least two binding domains, whereby one of said domains binds to human BCMA and a second domain binds to human CD3.

8. The bispecific binding agent of any one of the preceding claims, wherein the two binding domains are in identical or different formats.

9. The bispecific binding agent of claim 8, wherein
a) the first binding domain is an immunoglobulin single variable domain such as a VHH or a VH and the second binding domain is selected from a different immunoglobulin single variable domain, a BiTE or a diabody, or vice versa; or
b) the first binding domain is a full-size antibody and the second binding domain is an antibody fragment such as a diabody, or vice versa.

10. The bispecific binding agent of any one of the preceding claims, which binds to human BCMA and exhibits cross-reactivity with BCMA from one or more of monkey, mouse, rat, rabbit, pig and dog, wherein BCMA from monkey is preferably BCMA from cynomolgus or rhesus.

11. The bispecific binding agent of claim 10, wherein the bispecific binding agent cross-reacts with cynomolgus and mouse (Accession No. NP 035738) BCMA and recognizes an epitope in the region spanning amino acids 11-19 or 23-29 of human BCMA (Accession No. AB052772).

12. The bispecific binding agent of claim 10, wherein the bispecific binding agent cross-reacts with cynomolgus BCMA and recognizes an epitope spanning the region of amino acids 1-19, 23-29 or 32-51 of human BCMA (Accession No. AB052772).

13. A pharmaceutical composition containing at least one bispecific binding agent of any one of the preceding claims.

14. A bispecific binding agent of any one of claims 1 to 12 or a pharmaceutical composition of claim 13 for the treatment of plasma cell disorders or other B cell disorders that correlate with BCMA expression and for the treatment of autoimmune diseases.

15. A bispecific binding agent of any one of claims 1 to 12 or a pharmaceutical composition of claim 13 for the treatment of plasma cell disorders selected from plasmacytoma, plasma cell leukemia, multiple myeloma, macroglobulinemia, amyloidosis, Waldenstrom's macroglobulinemia, solitary bone plasmacytoma, extramedullary plasmacytoma, osteosclerotic myeloma, heavy chain diseases, monoclonal gammopathy of undetermined significance, smoldering multiple myeloma.

16. Anucleic acid molecule encoding the bispecific binding agent of any one of claims 1 to 12, wherein the bispecific binding agent is in the format of an antibody, an antibody-like molecule, a fragment or component thereof.

17. A host cell expressing or being capable of expressing one or more bispecific binding agents of any one of claims 1 to 12; and/or containing one or more nucleic acids of claim 16.

18. A method of manufacturing a bispecific binding agent of any one of claims 1-12, comprising the steps of:
- culturing host cells comprising one or more nucleic acids encoding the bispecific binding agent or a fragment thereof, under conditions that allow expression of the bispecific binding agent or a fragment thereof,
- optionally, in the case of distinct binding domain fragments, the assembly of such components, and
- recovering or isolating the bispecific binding agent expressed by the host cells from the culture.
